# EUROPEAN PATENT SPECIFICATION

(11) **EP 2 877 573 B1**
(45) Date of publication and mention of the grant of the patent: **21.09.2016**
(21) Application number: 13737206.6
(22) Date of filing: 10.07.2013
(51) Int. Cl.: C12N 9/00, A61K 8/66, A61Q 5/10, C12N 9/02

(54) **A METHOD OF PREPARING RECOMBINANT HUMAN TYROSINASE**
VERFAHREN ZUR HERSTELLUNG REKOMBINANTER MENSCHLICHER TYROSINASE
PROCÉDÉ DE PRÉPARATION DE TYROSINASE HUMAINE RECOMBINÉE

(30) Priority: 26.07.2012 EP 12177945; 17.09.2012 EP 12184662
(43) Date of publication of application: 03.06.2015
(73) Proprietor: Unilever N.V., 3013 AL Rotterdam (NL); Unilever PLC, London, Greater London EC4Y 0DY (GB)
(72) Inventor: CHANDRAMOWLI, Ganesh, Whitefield Bangalore 560 066 (IN); MAHAPATRA, Samiran, Whitefield Bangalore 560 066 (IN); THIMMAIAH, Sreenivasa, Whitefield Bangalore 560 066 (IN); DANDEKAR, Dineshkumar,Haribhau, Bangalore 560 076 Karnataka (IN); KRISHNAN, Seetha, Bangalore 560 099 (IN)
(74) Representative: Corsten, Michael Allan
(86) International application number: PCT/EP2013/064555
(87) International publication number: WO 2014/016118

(56) References cited:
- WO-A1-90/12869
- WO-A1-94/22468
- WO-A2-2011/060351
- M.Dolinska et al.: "Human modified tyrosinase and two temperature-sensitive mutants are soluble active enzymes", ARVO 2012 Abstract SEarch & Itinerary Builder, 7 May 2012 (2012-05-07), page Session No. 231, XP002688743, Retrieved from the Internet: URL:http://www.abstractsonline.com/Plan/Vi ewAbstract.aspx?mID=2866&sKey=856ee1ea-c6e 8-4af7-9cd1-cd022e45ca70&cKey=ec46eea5-b46 6-4cfb-87ef-a530779522ef&mKey={F0FCE029-9B F8-4E7C-B48E-9FF7711D4A0E} [retrieved on 2012-12-05]
- VAN OERS MONIQUE M: "Opportunities and challenges for the baculovirus expression system.", JOURNAL OF INVERTEBRATE PATHOLOGY JUL 2011, vol. 107 Suppl, July 2011 (2011-07), pages S3-15, XP028244442, ISSN: 1096-0805
- GEN-HUNG CHEN ET AL: "Expression of Recombinant Mature Human Tyrosinase from Escherichia coli and Exhibition of Its Activity without Phosphorylation or Glycosylation", JOURNAL OF AGRICULTURAL AND FOOD CHEMISTRY, AMERICAN CHEMICAL SOCIETY, US , vol. 60, no. 11 1 March 2012 (2012-03-01), pages 2838-2843, XP002694899, ISSN: 0021-8561, DOI: 10.1021/JF205021G Retrieved from the Internet: URL:http://pubs.acs.org/doi/abs/10.1021/jf 205021g [retrieved on 2012-02-21]
- W. LIU ET AL: "Molecular cloning and characterization of a laccase gene from the basidiomycete Fome lignosus and expression in Pichia pastoris", APPLIED MICROBIOLOGY AND BIOTECHNOLOGY, vol. 63, no. 2, 1 December 2003 (2003-12-01), pages 174-181, XP055075251, ISSN: 0175-7598, DOI: 10.1007/s00253-003-1398-0
- ENDO KOHKI ET AL: "Enzymological characterization of EpoA, a laccase-like phenol oxidase produced by Streptomyces griseus", JOURNAL OF BIOCHEMISTRY, JAPANESE BIOCHEMICAL SOCIETY / OUP, TOKYO; JP, vol. 133, no. 5, 1 May 2003 (2003-05-01), pages 671-677, XP002387763, ISSN: 0021-924X, DOI: 10.1093/JB/MVG086

## Description

### Field of the invention

The invention relates to a method of preparing enzymatically active recombinant human tyrosinase. The invention more particularly relates to a method that involves cloning and over expression of (recombinant) human tyrosinase using insect cells.

### Background of the invention

Tyrosinase is an enzyme known to be involved in melanin formation. Many skin lightening actives have been identified in the past by searching for safe and efficacious tyrosinase inhibitors. In carrying out *invitro* assays to determine tyrosinase inhibition and for studying enzyme kinetics, mushroom tyrosinase has been the most frequently used enzyme, while human and mouse melanocytic lysates have been used to a lesser extent. This is because mushroom tyrosinase is abundantly available, relatively inexpensive and easy to use. However mushroom tyrosinase is very different from human tyrosinase and it has been realized that using mushroom tyrosinase in such studies may lead to incorrect information about the potential of an active, as a skin lightening agent in humans. Thus, it is preferred that human tyrosinase is used. However there has been a problem in supply of human tyrosinase in sufficient quantities and with acceptable enzymatic activity. This has been overcome to some extent by using lysates from human melanocytes.

Human melanocytic lysate suffers from negatives due to its inherent complex nature of being a mixture of several proteins and therefore leading to unpredictable behavior when used in assays. Purification of tyrosinase results in low yield due to multi-step chromatographic processes. Thus, there exists a need for a better process to prepare human tyrosinase in high yield, superior purity and relevant enzymatic activity.

Commercial sources of recombinant human tyrosinase are available (e.g. Enzo Life Sciences) but the present inventors have found that its enzymatic activity is low, compared to equivalent amount of human melanocytic lysate. Therefore, it could not be effectively and reproducibly used as a suitable enzyme for carrying out screening of skin lightening actives. Truncated forms or fragments of human tyrosinase are available from other commercial sources but the present inventors have found them to be unsuitable in enzymatic assays. This is probably because many of them are bacterially expressed and lack glycosylation, critical for stability and activity. Thus bacterially expressed human tyrosinase, of which there are several publications in the literature, are found to be unsuitable for the purposes of enzymatic assays, which have to be sufficiently reproducible to enable efficient screening of actives for skin lightening.

WO90/12869 (Sloan Kettering) discloses a non-melanocytic eukaryotic cell constitutively expressing biologically active human tyrosinase. The present inventors have determined that the tyrosinase so expressed has to be prepared by breaking up fibroblasts and therefore the yield and purity, is comparatively poor.

Dolinska et al.: "Human modified tyrosinase and two temperature-sensitive mutants are soluble active enzymes", ARVO 2012 Abstract Search & Itinerary Builder, 7 May 2012 (2012-05-07), page Session No. 231, XP002688743 discloses the expression of a truncated human tyrosinase (residues 19 - 469) which was engineered as a synthetic codon optimized DNA sequence and cloned into baculovirus.

The present inventors have developed a method of preparing recombinant human tyrosinase where the products are easier to purify and exhibit biologically relevant enzymatic activity in a robust manner.

It is thus an object of the present invention to develop a method to prepare human tyrosinase in high yield and purity.

It is another object of the present invention to develop a method to prepare human tyrosinase in high yield and purity that is easy to scale up in order to prepare them in large quantities for commercial production.

It is yet another object of the present invention to develop a method to prepare human tyrosinase such that when used for enzyme assays one is able to obtain highly reproducible results.

### Summary of the invention

According to the present invention there is provided a method of preparing recombinant human tyrosinase comprising the steps of
(i) synthetically preparing a full length human tyrosinase gene defined as extending from the corresponding protein's N-terminus to its amino acid 473 or higher in a codon optimized manner using PCR;
(ii) cloning said gene in to a baculovirus expression system;
(iii) preparing bacmids from said clones;
(iv) infecting an insect cell with said bacmids to prepare a virus titer; and
(v) optimizing MOI (multiplicity of infection) and time points for expression screening of the desired human tyrosinase.

It is particularly preferred that the cell media of step (v) includes a copper salt.

### Detailed description of the invention

These and other aspects, features and advantages will become apparent to those of ordinary skill in the art from a reading of the following detailed description and the appended claims. For the avoidance of doubt, any feature of one aspect of the present invention may be utilised in any other aspect of the invention. The word "comprising" is intended to mean "including" but not necessarily "consisting of" or "composed of." In other words, the listed steps or options need not be exhaustive. It is noted that the examples given in the description below are intended to clarify the invention and are not intended to limit the invention to those examples per se. Similarly, all percentages are weight/weight percentages unless otherwise indicated. Except in the operating and comparative examples, or where otherwise explicitly indicated, all numbers in this description indicating amounts of material or conditions of reaction, physical properties of materials and/or use are to be understood as modified by the word "about". Numerical ranges expressed in the format "from x to y" are understood to include x and y. When for a specific feature multiple preferred ranges are described in the format "from x to y", it is understood that all ranges combining the different endpoints are also contemplated. The disclosure of the invention as found herein is to be considered to cover all embodiments as found in the claims as being multiply dependent upon each other irrespective of the fact that claims may be found without multiple dependency or redundancy.

The present invention relates to preparing recombinant human tyrosinase using insect cells. This method has the advantage that the tyrosinase thus prepared has excellent and visibly apparent enzymatic activity. The disclosed process for preparing the human tyrosinase has the following useful applications. The method is useful to directly mass produce the human tyrosinase. Further, this material has applications in the area of including tyrosinase in formulations to darken skin and hair and for alleviating hypopigmentary conditions like vitiligo, patchy (hypo)pigmentation, hypo-pigmented spots and for helping achieve uniform skin color. It is also envisaged for use in the area of sunless tanning. The human tyrosinase produced by the method of the present invention can be used for development of commercial scale assays for identification of skin lightening actives. Additionally the method can be extended to *in vitro* production of melanin and its variants. Yet another advantageous benefit of the present invention is in the design of molecular kits e.g. in the area of diagnostics, and vaccine development.

The detailed steps that were carried out for preparing recombinant human tyrosinase in developing the present invention are the following:
(i) Synthetically preparing in a codon optimized manner, a full length human tyrosinase gene using PCR. The full length human tyrosinase gene as per the present invention is defined as a human tyrosinase gene whose corresponding protein extends from the N-terminus to the amino acid 473 or higher. It is possible by way of the present invention to use the full length human tyrosinase gene from alternate sources other than human sequence.

The presence of the membrane spanning domain in full length tyrosinase makes it a trans-membrane protein. The expression pattern, solubility and other biochemical parameters of this class are very different from soluble proteins e.g. the truncated human tyrosinase hTyrCtr residues 19 to 469 used in Dolinska et al. cited hereinabove. It thus continues to be a challenge to produce the desired tyrosinase in an enzymatically active form starting with the full length human tyrosinase gene. This challenging problem has been overcome by way of the present invention.
(ii) The gene thus prepared, which is the full length version is then cloned into a eukaryotic baculovirus expression vector system. The eukaryotic vector is preferably a pFastbac™ vector. The gene is preferably cloned in the pFastbac™ vector between the EcoRI and HindIII sites. The cloned genes are confirmed/ verified by regular DNA sequencing.
(iii) Bacmids are then prepared from the clones.
(iv) The Bacmids are then used to infect an insect cell to prepare a virus titer. The preferred insect cells are Sf9 or High Five. It is preferred that the resultant P1 virus titer are further passaged through the bacmids to obtain further titers e.g. P2 and P3 titers. Preferred aspects provides for the P3 virus to be used to infect the HighFive™ and Sf9 cells to express the recombinant human tyrosinase (which is full length depending on the starting gene).

The infected insect cells are then optimized with the above method of multiplicity of infection and at various time points for expression screening of the desired human tyrosinase by western blot.

When the full length human tyrosinase gene is used, it is preferred that a copper salt is included in the cell media of step (v). Preferred copper salts are copper chloride or copper sulphate. A further preferred aspect includes mixtures of different copper salts. When included, the copper salt is present at a concentration in the range of 0.1 to 25 micromoles per litre of the cell media.

In actual practice, the following steps were carried out to evaluate the efficacy of the method of the present invention.

Standard western blot procedure was used to examine the extent of tyrosinase protein expression as well as determine optimal time point to harvest the cells. For scale up work, it was decided to use HighFive™ cells further. 100 ml scale growth of "infected" HighFive™ cells were carried out and both the spent media as well as the cell pellets were collected. The latter was lyzed by regular sonication method and the activity of the tyrosinase enzyme in various samples were tested using the typical DOPA oxidation and DOPA+MBTH coupling assays.

In a preferred aspect when the full length human tyrosinase gene is used, the method comprises the step of lysing the insect cell to prepare the human tyrosinase.

Collecting the human tyrosinase from the insect cell growth media supernatant is preferred.

The full length tyrosinase present in the lysate and the spent media in case of truncated tyrosinase case were found to exhibit robust enzymatic activity. Thus the enzymatic activity assay and the western blot were together used to demonstrate the successful production of two different versions of recombinant human tyrosinase in an active form.

Illustrative is a method of colouring substrates comprising the step of contacting the substrate with a melanin precursor and human tyrosinase prepared using the method of this invention.

Also illustrative is a composition to colour a substrate comprising (i) a melanin precursor; (ii) human tyrosinase prepared using a method of the invention; and (iii) a cosmetically acceptable base. The cosmetically acceptable base is preferably a cream, lotion or gel.

In the method and the composition above, the substrate is preferably hair more preferably graying human hair. The melanin precursor used is preferably tyrosine or dihydoxy phenyl alanine (DOPA).

### Summary of the Figures

Figure 1: The synthetic gene sequence codon optimized for baculovirus expression. This gene sequence had the same protein sequence as human tyrosinase.
Figure 2: Visual examination (prior to cell lysis by sonication) of the samples of cell pellet obtained in the case of insect cells expressing Full (FL) rHuTyrase (Example 1) as compared to the control uninfected cells (Example A).

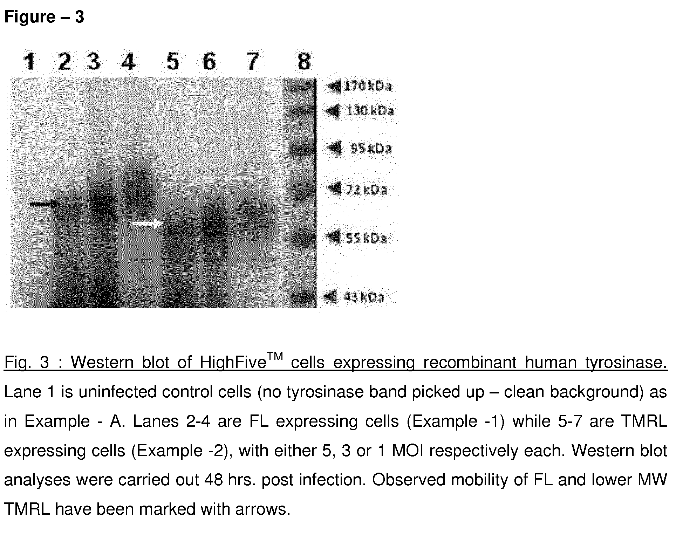

Figure 3: Depicts the extent of recombinant human tyrosinase expression in Sf9 and HighFive™ cell lines, as judged by tyrosinase protein western blot.

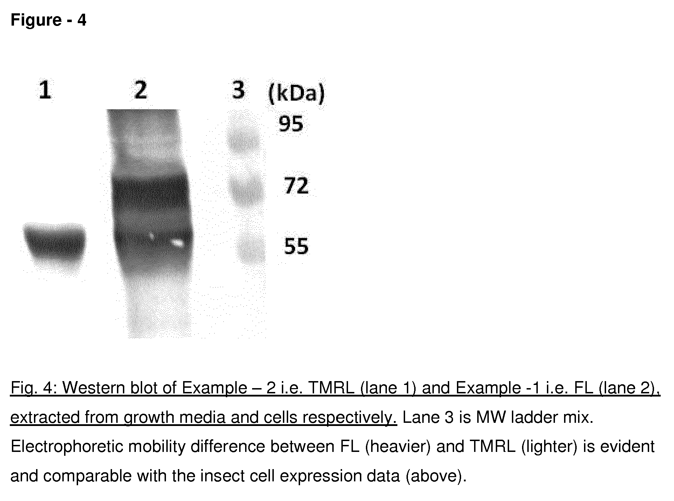

Figure 4: Depicts the Western blot of Example 2 i.e. Transmembrane Region less (TMRL) (lane 1) and Example 1 i.e. FL (lane 2), extracted from growth media and cells respectively.

The invention will now be demonstrated by way of the following non-limiting examples.

### Examples

### Examples A, 1 and 2: Method of preparation of human tyrosinase as per the

### invention (Example 1 and 2) compared to control (Example A)

The samples of Example 1 and 2 and Example A were prepared as per the following procedure.

### Materials used

L-Tyrosine (numbered T3754), L-dihydoxy phyenylalanine (L-DOPA numbered D9628), 4-ethyl resorcinol (4- ER numbered E4820-0) and 3-methyl-2-benzothiazolinone (MBTH numbered 12973-9) were obtained from Sigma/Aldrich. Instruments used to measure optical density (OD) were the Nanodrop 2000c spectrophotometer (Thermo Scientific), TECAN GeniosPro and Infinite M1000 multi-well plate readers. Data was analyzed and presented using SigmaPlot software (ver. 10.0).

### Preparation of Synthetic Human Tyrosinase Gene (full length & truncated), Codon Optimized for Insect Cell Expression

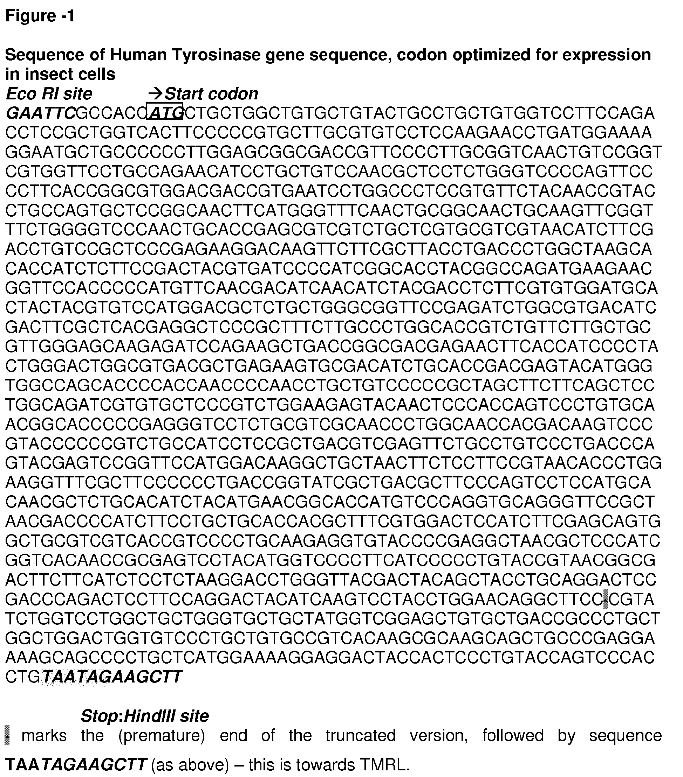

Full Length (FL) rHuTyrase gene [isoform 1 of P14679 (TYRO_HUMAN) Reviewed, UniProtKB/Swiss-Prot], with Eco RI and Hin dill ends was synthesized (Example 1). The synthetic gene had the same protein sequence as human tyrosinase but gene sequence was codon optimized for baculovirus expression. Chemical synthesis of the gene was preferred over human melanocytes: cDNA route, from the angle of exact gene sequence. Sequence detail is given in Figure - 1. Truncated version (TMRL- Trans membrane region less) (Example 2) version was designed as follows. TMR in huTyrase protein sequence was predicted using online tools, DAS, HMMTOP, SOSUI, TMHMM and MOBYLE. As a consensus, amino acid residue 476 was identified as the 1 st residue in TMR. As a result, TMRL construct was terminated at 472nd amino acid residue, Serine (...LEQAS), ahead of the start of TMR. Thus, TMRL version lacks both the TMR as well as the cytoplasmic tail of FL.
TMRL was prepared by PCR, using synthetic FL rHuTyrase gene template (shown in Figure 1). Primers used for that PCR were:
Tyro-fwd-EcoRI: 5'-TGTATATGAATTCGCCACCATGCTGCTGGCTGTGCTG-3' Tyro-trunc-H*ind*III: 5'-
TTAAGTAAGCTTCTATTAGGAAGCCTGTTCCAGGTAGGAC-3'.

### PCR of truncated human tyrosinase from full length version

TMRL gene was prepared by regular PCR methods, using FL version as the template.

The overall strategy for cloning (both FL & TMRL) is below:
- Tyrosinase genes were end-trimmed with *Eco* RI and *Hind* III restriction enzymes and ligated to similarly digested but additionally, de-phosphorylated pFASTBAC™ vector(Invitrogen), using T4 DNA Ligase at 25°C for 16 hrs.
- Ligated products were transformed into *E. coli* Omnimax competent cells and plated on LB ampicillin plates. Plates were incubated at 37°C for 16h
- Colonies were screened by PCR using vector specific and gene specific primers and positive colonies were inoculated for plasmid isolation
- Colony PCR products were resolved in 1 % agarose gel.
- Positive clones identified by colony PCR were propagated.
- Plasmid isolation was carried out using Sigma miniprep column, and subjected to restriction digestion by *Eco* RI and *Hind* III to confirm insert release (~1.6 kbp)
- Plasmid clones were confirmed by sequencing with vector and internal gene specific primers.

TMRL construct lacks two critical Serine residues (Ser505 & Ser509), due to the absence of the normal cytoplasmic C-terminal tail present in the FL version. Both these residues are phosphorylated by PKCβ and results in activation of tyrosinase. It is to be appreciated that intricate regulation of enzymatic activity could be different under certain conditions. The full length construct has this domain intact and useful from that perspective too.

### Preparation of Bacmids

- Sequence confirmed clones of tyrosinase cloned in pFastBac™ were transformed into DH10 BAC competent cells
- The regeneration mixes were plated on LB tetracycline (10 µg/ml), kanamycin (50 µg/ml), gentamycin (7 µg/ml), X-gal and IPTG plates and incubated at 37°C for 16 hrs.
- White colonies were selected and patched onto the LB Tet/Kan/Gen/IPTG/X-gal plates and incubated at 37°C for 16 hrs.
- White colonies were further screened by colony PCR to confirm the orientation of the gene.
- PCR products were checked in 1 % agarose gel.
- PCR positive clones were inoculated for Bacmid isolation.
- 2 ml of overnight culture were used for Bacmid isolation by modified alkaline lysis method.
- Bacmid preparations were quantified and verified again by PCR with vector and gene specific primes as mentioned earlier.

### Culturing of Sf9 and High Five cells & Transfection

- Sf9 cells (Invitrogen) were maintained in Sf900III SFM medium to obtain logarithmic phase culture at cell density of 1.5 - 2 million cells/ml.
- High Five cells (Invitrogen) were maintained in Express Five SFM medium to obtain logarithmic phase culture at cell density like above.
- Transfection of log phase Sf9 culture using Cellfectin II (Invitrogen) with Fulllength (FL) Tyr and Truncated Tyr (TMRL) bacmid constructs in 6-well plates with 3 µg bacmid DNA and 8 µl Cellfection reagent.
- Sf9 cells were seeded at a density of 8X10⁵ cells/ml per well of a 6 well plate
- Post seeding, the plates were incubated at room temperature in the hood for the cells to attach.
- For each transfection sample, complexes were prepared as follows:
   ∘ 8 µl of Cellfectin II was mixed gently in 100 µl Grace's unsupplemented media (without antibiotics and serum);
   ∘ 1 µg bacmid DNA was diluted in 100 µl Grace's unsupplemented media (without antibiotics and serum) and mixed gently;
   o diluted DNA was mixed with diluted Cellfectin II (total volume~210 µl) and incubated for 15-30 minutes at room temperature;
   o ~210 µl DNA-lipid mixture or transfection mixture was added dropwise onto the cells and incubated at 27°C for 4-5 hrs.
- Post incubation, the transfection mixture was removed and replaced with 2 ml of complete growth medium (Grace's supplemented media with 10% FBS). The cells were incubated at 27°C for 72 hours, until signs of viral infection were observed.

### Preparation of viral stocks

### • P1 viral stock

- for both the bacmid constructs (FL and TMRL), spent medium was harvested, centrifuged to remove the cell debris and stored at 4°C. This stock was denoted as P1 baculovirus which was further amplified as described below.

### • Amplification of recombinant P1 baculovirus stock to generate P2 & P3 viral stocks

- P1 baculovirus stock from both construct were amplified at a 50 ml scale to generate P2 baculovirus
   o log phase Sf9 cells in shake flask at ~ 1 million cells/ml (50 ml) were infected with 5 ml of P1 baculovirus stock (assuming P1 titer of 1 million pfu/ml)
   o baculovirus were harvested at 60% cell viability (96 - 120 hrs. post-infection).
- P3 viral stock was generated using P2 viral stock by same method.

### • Determination of P3 virus titer by BacPAK Baculovirus Rapid Titer Kit

- Reagents were prepared as per BacPAK Baculovirus Rapid Titer Kit (Clontech)
- Sf9 cells were seeded at a density of 6.5 x 10⁴ cells/well in 96 well plate and incubated at 27°C for 1 hrs.
- cells were infected with virus samples (pooled from both shake flasks) at 10⁻³, 10⁻⁴, 10⁻⁵ dilutions
- viral antigen released after infection were determined using supplied immunoassay reagents as per protocol provided in the kit
- stained foci of infection (Blue color clusters) were counted using light microscopy and used to calculate viral titer in the amplified stock
- P3 baculovirus titers were - FL : 3.4X10⁷ pfu/ml & TMRL : 5.2 X10⁷ pfu/ml.

### Infection of Sf9 and HighFive™ cells with baculovirus carrying FL and TMRL

- Log phase cultures of HighFive™ and Sf9 cells (1 million cells/ml, 20 ml in 150 ml Erlenmeyer flasks) were infected with P3 baculovirus at MOIs (multiplicity of infection) of 1, 3 and 5.
- Cells were harvested and pelleted at 24 hrs., 48 hrs., and 72 hrs. post-infection.
- Both Sf9 and High-Five cells appeared completely infected and viability values were much reduced as compared to the respective controls.
- ∼10 million cells were processed for analysis by western blot.

### Western Blot for analysis of expression of full length and truncated Tyrosinase

- The cell pellets were dissolved in 500 µl of 1X Laemmli buffer, kept in ice water bath and sonicated for 15 cycles (56% amplitude, 2 sec. ON 3sec. OFF cycle).
- Following lysis, samples were denatured by boiling for 5 min. and denatured samples centrifuged @ 10,000 rpm/2 min.
- 12.5 µl of each sample's supernatant loaded onto 10% SDS PAGE gel along with PageRuler™ Prestained Protein Ladder (#SM0671, Fermentas) and resolved electrophoretically.
- Transfer from SDS-PAGE gel onto Nitrocellulose membrane was carried out in a submerged transfer system (Biorad) for 2 hrs. in 4°C @100V.
- The transferred membrane then blocked with 5% skimmed milk in 1x PBS @ RT for 1 hr.
- The blot was incubated with primary antibody - 1:500 dilution (mouse antihuman tyrosinase) in 1xPBS containing 1% BSA, overnight at 4°C.
- Following incubation, the blot was washed with 1X PBS containing 0.05% Tween-20 for three times.
- The blot was then incubated for two hours at room temperature with 1:10³ diluted secondary antibody - horse anti-mouse antibody HRP-Linked (Cell Signaling Technology) diluted in 1xPBS containing 1% BSA.
- The blot was then rinsed thrice with 1X PBS containing 0.05% Tween-20, developed using ECL kit (Amersham Biosciences) and documented using the Bio-Rad Chemidoc XRS Gel documentation System and Quantity One Software.

### Preparation of Lysates from Baculovirus Infected Insect Cells

- This was carried out by sonication (ultrasonic homogenizer) on ice-water bath
   ∘ 30-35% working power in Bandelin Sonoplus HD2070 sonicator;
   o sonication cycle - 5 sec. ON; OFF and mix on ice; 4-5 such cycles.
- Sonicated materials were centrifuged at 10,000xg/4°C/15 min. and the clarified supernatant immediately separated from the pellet/debris.
- After saving a small aliquot, rest of TMRL growth media supernatant was concentrated ~30x through a 20kDa Pierce centrifugation filter.
- In all cases, protein content was estimated by the regular Bradford method.

### Western Blot, post lysis of Insect cells expressing recombinant human Tyrosinase

- Protein concentration was estimated by Bradford Assay and 20 µg protein samples each were separated through a 12% SDS-PAGE gel by electrophoresis
- The gel was removed from the cassette and soaked in transfer buffer (25 mM Tris, 192 mM Glycine, 20% Methanol) for about 10 min. Simultaneously, the transfer membrane (PVDF) was separately wet thoroughly with transfer buffer, after its presoak in 100% Methanol
- Proteins were wet transferred from gel to PVDF membrane (1 hr., 4 °C) by using regular western blot apparatus (Amersham Biosciences, Cat.# 80-6204-26) and then incubated the PVDF membrane in blocking solution (5% skimmed milk in 1x PBS) for 1 hr.
- The blot membrane was removed from blocking solution and incubated overnight (at 4 °C) in a solution of 1:500 diluted mouse anti- human Tyrosinase antibody (Santacruz, Cat.No.SC-20035) in 1X PBS containing 1% BSA. The next morning, washed the membrane thrice for for10 min. in 1X PBS containing 0.05% Tween20
- The blot was then incubated in 1:2000 diluted solution of goat anti-mouse secondary antibody (Santacruz,Cat No.SC2047) in1X PBS for 60 min at 37 °C
- The blot membrane was once again washed thrice for 10 min. in 1X PBS containing 0.05% Tween20 and then developed using the substrate solution (0.02% w/v NBT + 0.03% w/v BCIP in 10 mM Tris-MgCl2 buffer pH 9.5) at 37 °C till bands were visible to eye.
- To prevent over development, the blot was immediately washed several times in water, dried and photographed.

### In-Gel Analyses of Tyrosinase Enzymatic Activity

Electrophoresis was carried out through a 10% SDS-PAGE gel but tested samples were prepared in regular SDS-PAGE gel loading buffer in the absence of reducing agent (β-Mercaptoethanol or DTT) and were not boiled.

After completion of electrophoresis, the gel was washed extensively with water, incubated in 50 mM phosphate pH 6.8 buffer for a few minutes and then transferred into 2 mM DOPA solution or (0.5 mM Tyrosine + 15 µM DOPA) in the same buffer. Reactions were carried out with gentle shaking at 37°C for ~ 30 min., visualized directly and photographed.

### Solution Phase Assays for Tyrosinase Enzymatic Activity

DOPA oxidation activity was examined by a slightly modified version of the procedure earlier reported by Harris & Winder [Eur. J. Biochem. (1991) 198: 317-326], excluding Perchloric acid and Dimethylformamide but capturing a picture on reaction progress. Separately, *in vitro* melanin formation assay was carried out for ~ 7 hrs. at 37 °C, using a substrate mix (of 0.5 mM L-Tyrosine + 15 µM L-DOPA) in 50 mM phosphate buffer (pH 6.8) and using indicated amounts of protein. This assay covers both tyrosine hydroxylation and DOPA oxidation activities together in the same run. Human melanocytic lysate served as the reference positive control and absence of any protein served as the no enzyme negative control (uncatalyzed background reaction) for these reactions. Assay volumes of 0.1 ml were adequate, indicating scalability for enhanced high throughput screening purposes.

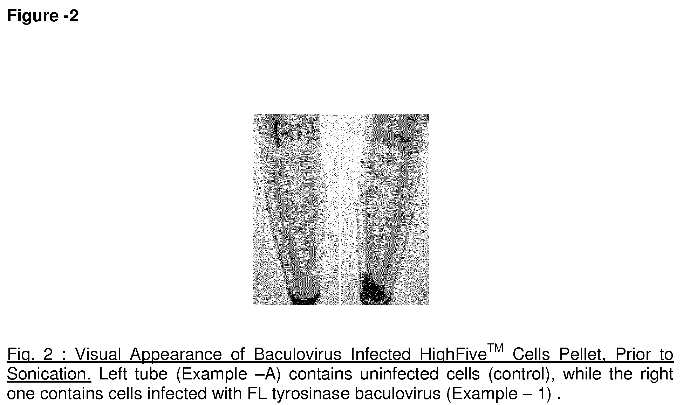

Visual examination (prior to cell lysis by sonication) of the samples (See Figure 2) revealed that the cell pellet obtained in the case of insect cells expressing FL rHuTyrase (Example 1) was black (ascribed to formation of dark melanin like pigment within the cells) while the control uninfected cells (Example A) were pale in colour.

### Examples 3 to 5 and B to E (DOPA+MBTH assay): Solution phase tyrosinase

### enzymatic assay

The samples used were:
Example B: HML (human melanocytic lysate; prepared by sonicating pigmenting human primary melanocytes in a manner similar to sonication of HighFive™ insect cells) which is a positive control sample.
Example C: NEC (No enzyme control) which is a negative control sample.
Example 3: Sonication lysate supernatant of HighFive™ cells expressing FL tyrosinase.
Example 4: Sonication lysate supernatant of HighFive™ cells expressing TMRL tyrosinase.
Example 5: Growth media supernatant in which TMRL cells were grown. Example D: Sonication lysate supernatant of control uninfected HighFive™ cells. Example E: Growth media supernatant in which above cells were grown Reaction samples were photographed and converted into gray scale intensity (GSI) by computer software digitization. Numerical differences (dᵢ) were calculated between GSI of any sample and that of reference control background sample (Example D). dₘₐₓ was the maximal difference, that between HML (positive reference control, Example B) and the reference control background sample (Example D). A ratio scale was then calculated as (dᵢ/dₘₐₓ) and results tabulated (Table 1); 0 indicates a transparent sample while ratio closer to 1 indicates activity comparable to HML.

**Table 1: DOPA + MBTH Reaction**

| **Example** # | **Material** | **Scale ratio dᵢ/dₘₐₓ** |
|---|---|---|
| B | HML | 1.00 |
| C | NEC | 0.00 |
| 3 | FL Lysate | 0.85 |
| 4 | TMRL lysate | 0.11 |
| 5 | TMRL growth media supernatant | 0.53 |
| D | Untransfected cells lysate | 0.06 |
| E | Untransfected cells media | 0.00 |

The data in Table 1 above indicates that samples prepared as per the invention (Examples 3 to 5) exhibit excellent level of enzymatic activity (clearly visible to the eye).

### Examples 6, 7, F and G

When the activity of 15 µg protein equivalent recombinant human tyrosinase samples were tested in DOPA (2 mM) assay (in absence of MBTH), OD_{450 nm} values reached after 90 min. (indication of extent of oxidation) were converted into a ratio form and are tabulated below:

**Table 2: Extent of DOPA oxidation by Recombinant Human Tyrosinase**

| **Sample** | **Scale ratio dᵢ/dₘₐₓ** |
|---|---|
| Example F: No Enzyme negative Control | 0.00 |
| Example G: Commercially sourced *E. coli* (bacteria) expressed recombinant human tyrosinase | 0.04 |
| Example 6 (FL lysate supernatant) | 0.90 |
| Example 7 (TMRL growth media supernatant) | 1.00 |

While both FL (Example 6) and TMRL (Example 7) are active, the latter is more so at the same protein equivalent. In part, this can be attributed to substantial cellular proteins present along with FL in the lysate, while contaminants in case of TMRL is comparatively less as it has been secreted out into growth medium. Thus, TMRL offers an easier scale up and purification option directly, without compromising on tyrosinase enzymatic activity.

### Examples 8 to 12 and Example H: Effect of known tyrosinase inhibitors on human tyrosinase prepared by the invention, in the in vitro Tyrosine to melanin assay

Example H: NEC negative control (no protein).
Example 8: 15 µg sample of human tyrosinase prepared using truncated gene (TMRL).
Examples 9 and 10: 30 and 60 µg samples of TMRL.
Example 11; Sample of example 10 inhibited by using 50 mM Arbutin.
Example 12; Sample of example 11 inhibited by using 25 mM Kojic acid.

At the completion of the reaction, samples were photographed and data analyzed further in the same manner described under the DOPA+MBTH section. The data is summarized in Table - 3 below.

**Table 3: Tyrosinase activity of TMRL inhibited by known tyrosinase inhibitors**

| **Example #** | **Material** | **Scale ratio dᵢ/dₘₐₓ** |
|---|---|---|
| H | NEC | 0.00 |
| 8 | TMRL (15 µg) | 0.41 |
| 9 | TMRL (30 µg) | 0.86 |
| 10 | TMRL (60 µg) | 1.00 |
| 11 | TMRL (60 µg) + 50mM Arbutin | 0.00 |
| 12 | TMRL (60 µg) + 25mM Kojic acid | 0.00 |

The data in Table 3 indicates the extent of darkening obtained with samples of the invention (Examples 8 to 10), while these samples do not develop colour in presence of known tyrosinase inhibitors (Examples 11-12).

### Examples 13 to 16: Effect of using copper salts

Experiments as per Example 2 were conducted additionally including a copper salt as shown in Table 4. The samples were analysed in a manner as used for generating data of Table 1 and the scale ratio with respect to the best sample (Example 16) is summarized in Table 4. For comparision, scale ratio for Example 7 is recalculated in the same way i.e. with respect to the OD of the best sample when copper salt is used (Example 16).

**Table 4**

| **Example** | **Copper salt (concentration in micromoles/liter)** | **Scale ratio (510 nm)** |
|---|---|---|
| 7 | No copper salt | 0.05 |
| 13 | Copper chloride (0.5) | 0.35 |
| 14 | Copper chloride (5.0) | 0.50 |
| 15 | Copper sulphate (0.5) | 0.31 |
| 16 | Copper sulphate (5.0) | 1.0 |

The data in Table 4 indicates the improved efficacy of the method of the invention when a copper salt is used in the cell media during protein expression.

The invention thus provides for a method to prepare human tyrosinase in high yield, easy to scale up for commercial production and purification and be used for enzyme assays, to obtain highly reproducible results.

## Claims

1. A method of preparing recombinant human tyrosinase comprising the steps of
(i) synthetically preparing a full length human tyrosinase gene defined as extending from the corresponding protein's N-terminus to its amino acid 473 or higher in a codon optimized manner using PCR;
(ii) cloning said gene in to a baculovirus expression system;
(iii) preparing bacmids from said clones;
(iv) infecting an insect cell with said bacmids to prepare a virus titer; and
(v) optimizing MOI (multiplicity of infection) and time points for expression screening of the desired human tyrosinase.

2. A method as claimed in claim 1 wherein the cell media of step (v) includes a copper salt.

3. A method as claimed in claim 1 or 2 wherein said copper salt is copper chloride or copper sulphate.

4. A method as claimed in claim 2 or 3 wherein said copper salt is included at a concentration in the range of 0.1 to 25 micromoles per litre of the cell media.

5. A method as claimed in claim 1 wherein, the method comprises the step of lysing said insect cell to prepare the human tyrosinase.

6. A method as claimed in claim 3 or 4 comprising the step of lysing said insect cell to prepare the human tyrosinase or collecting the human tyrosinase from the insect cell growth media supernatant.

7. A method as claimed in any one of the preceding claims wherein said gene in step (ii) is cloned onto a eukaryotic vector.

8. A method as claimed in claim 7 wherein said eukaryotic vector is a baculovirus expression vector.

## Patentansprüche

1. Verfahren zur Herstellung rekombinanter humaner Tyrosinase, umfassend die Schritte
(i) synthetisches Herstellen eines humanen Tyrosinase Gens mit voller Länge, definiert als vom N-terminus des zugehörigen Proteins bis zu seiner Aminosäure 473 oder höher reichend, in codon-optimierter Weise unter Verwendung von PCR;
(ii) Klonieren des Gens in ein Baculovirus-Expressionssystem;
(iii) Herstellen von Bacmiden aus den Klonen;
(iv) Infizieren einer Insektenzelle mit den Bacmiden, um einen Virustiter herzustellen; und
(v) Optimieren der MOI (Multiplizität der Infektion) und Zeitpunkte für Expressionsscreenings der gewünschten Tyrosinase.

2. Verfahren gemäß Anspruch 1, wobei das Zellmedium von Schritt (v) ein Kupfersalz beinhaltet.

3. Verfahren nach Anspruch 1 oder 2, wobei das Kupfersalz Kupferchlorid oder Kupfersulfat ist.

4. Verfahren nach Anspruch 2 oder 3, wobei das Kupfersalz in einer Konzentration im Bereich von 0,1 bis 25 Mikromol pro Liter in dem Zellmedium enthalten ist.

5. Verfahren nach Anspruch 1, wobei das Verfahren den Schritt der Lyse der Insektenzellen, um humane Tyrosinase herzustellen, umfasst.

6. Verfahren nach Anspruch 3 oder 4 umfassend den Schritt der Lyse der Insektenzellen, um humane Tyrosinase herzustellen, oder Sammeln der humanen Tyrosinase aus dem Überstand des Wachstumsmediums der Insektenzellen.

7. Verfahren nach irgendeinem der vorhergehenden Ansprüche, wobei das Gen in Schritt (ii) in einen eukaryotischen Vektor kloniert wird.

8. Verfahren nach Anspruch 7, wobei der eukaryotische Vektor ein Bakulovirus Expressionsvektor ist.

## Revendications

1. Procédé de préparation d'une tyrosinase humaine recombinante comprenant les étapes consistant à
(i) préparer de manière synthétique un gène de tyrosinase humaine de pleine longueur défini comme s'étendant à partir de l'extrémité N-terminale de la protéine correspondante jusqu'à son acide aminé 473 ou plus d'une manière optimisée en codons en utilisant une PCR ;
(ii) cloner ledit gène dans un système d'expression de baculovirus;
(iii) préparer des bacmides à partir desdits clones;
(iv) infecter une cellule d'insecte avec lesdits bacmides afin de préparer un titre de virus ; et
(v) optimiser la MOI (multiplicité d'infection) et les moments pour cribler l'expression de la tyrosinase humaine souhaitée.

2. Procédé selon la revendication 1, dans lequel le milieu cellulaire de l'étape (v) comprend un sel de cuivre.

3. Procédé selon la revendication 1 ou 2, dans lequel ledit sel de cuivre est le chlorure de cuivre ou le sulfate de cuivre.

4. Procédé selon la revendication 2 ou 3, dans lequel ledit sel de cuivre est inclus à une concentration dans la plage de 0,1 à 25 micromoles par litre du milieu cellulaire.

5. Procédé selon la revendication 1, où le procédé comprend l'étape de lyse de ladite cellule d'insecte afin de préparer la tyrosinase humaine.

6. Procédé selon la revendication 3 ou 4, comprenant l'étape de lyse de ladite cellule d'insecte afin de préparer la tyrosinase humaine ou de collecter la tyrosinase humaine à partir du surnageant du milieu de croissance de la cellule d'insecte.

7. Procédé selon l'une quelconque des revendications précédentes, dans lequel ledit gène à l'étape (ii) est cloné sur un vecteur eucaryote.

8. Procédé selon la revendication 7, dans lequel ledit vecteur eucaryote est un vecteur d'expression baculovirus.


### SEQUENCE LISTING

<110> Unilever N.V.
<120> A METHOD OF PREPARING RECOMBINANT HUMAN TYROSINASE
<130> G2125
<140> EP12177945.8
   <141> 2012-07-26
<160> 3
<170> PatentIn version 3.5
<210> 1
   <211> 37
   <212> DNA
   <213> Artificial Sequence
<220>
   <223> primer
<400> 1
   tgtatatgaa ttcgccacca tgctgctggc tgtgctg 37
<210> 2
   <211> 40
   <212> DNA
   <213> Artificial Sequence
<220>
   <223> primer
<400> 2
   ttaagtaagc ttctattagg aagcctgttc caggtaggac 40
<210> 3
   <211> 1611
   <212> DNA
   <213> Artificial Sequence
<220>
   <223> synthetic gene sequence of Human Tyrosinase, codon optimized for baculovirus expression
<400> 3
   gaattcgcca ccatgctgct ggctgtgctg tactgcctgc tgtggtcctt ccagacctcc      60
   gctggtcact tcccccgtgc ttgcgtgtcc tccaagaacc tgatggaaaa ggaatgctgc     120
   cccccttgga gcggcgaccg ttccccttgc ggtcaactgt ccggtcgtgg ttcctgccag     180
   aacatcctgc tgtccaacgc tcctctgggt ccccagttcc ccttcaccgg cgtggacgac     240
   cgtgaatcct ggccctccgt gttctacaac cgtacctgcc agtgctccgg caacttcatg     300
   ggtttcaact gcggcaactg caagttcggt ttctggggtc ccaactgcac cgagcgtcgt     360
   ctgctcgtgc gtcgtaacat cttcgacctg tccgctcccg agaaggacaa gttcttcgct     420
   tacctgaccc tggctaagca caccatctct tccgactacg tgatccccat cggcacctac      480
   ggccagatga agaacggttc cacccccatg ttcaacgaca tcaacatcta cgacctcttc      540
   gtgtggatgc actactacgt gtccatggac gctctgctgg gcggttccga gatctggcgt      600
   gacatcgact tcgctcacga ggctcccgct ttcttgccct ggcaccgtct gttcttgctg      660
   cgttgggagc aagagatcca gaagctgacc ggcgacgaga acttcaccat cccctactgg      720
   gactggcgtg acgctgagaa gtgcgacatc tgcaccgacg agtacatggg tggccagcac      780
   cccaccaacc ccaacctgct gtcccccgct agcttcttca gctcctggca gatcgtgtgc      840
   tcccgtctgg aagagtacaa ctcccaccag tccctgtgca acggcacccc cgagggtcct      900
   ctgcgtcgca accctggcaa ccacgacaag tcccgtaccc cccgtctgcc atcctccgct      960
   gacgtcgagt tctgcctgtc cctgacccag tacgagtccg gttccatgga caaggctgct     1020
   aacttctcct tccgtaacac cctggaaggt ttcgcttccc ccctgaccgg tatcgctgac     1080
   gcttcccagt cctccatgca caacgctctg cacatctaca tgaacggcac catgtcccag     1140
   gtgcagggtt ccgctaacga ccccatcttc ctgctgcacc acgctttcgt ggactccatc     1200
   ttcgagcagt ggctgcgtcg tcaccgtccc ctgcaagagg tgtaccccga ggctaacgct     1260
   cccatcggtc acaaccgcga gtcctacatg gtccccttca tccccctgta ccgtaacggc     1320
   gacttcttca tctcctctaa ggacctgggt tacgactaca gctacctgca ggactccgac     1380
   ccagactcct tccaggacta catcaagtcc tacctggaac aggcttcccg tatctggtcc     1440
   tggctgctgg gtgctgctat ggtcggagct gtgctgaccg ccctgctggc tggactggtg     1500
   tccctgctgt gccgtcacaa gcgcaagcag ctgcccgagg aaaagcagcc cctgctcatg     1560
   gaaaaggagg actaccactc cctgtaccag tcccacctgt aatagaagct t              1611